# EUROPEAN PATENT APPLICATION

(11) **EP 4 287 197 A1**
(43) Date of publication of application: **06.12.2023**
(21) Application number: 22176035.8
(22) Date of filing: 30.05.2022
(51) Int. Cl.: G16H 20/10

(54) **COMPUTER IMPLEMENTED METHOD FOR DETERMINING A DRUG DOSE, TRAINING METHOD AND SYSTEM**

(71) Applicant: BIOTRONIK SE & Co. KG, 12359 Berlin (DE)
(72) Inventor: Diem, Björn Henrik, 14197 Berlin (DE); Linnemann, Antje, 12359 Berlin (DE); Reich, Anastasia, 12524 Berlin (DE)
(74) Representative: Biotronik Corporate Services SE

(57) **Abstract**

The invention relates to a computer implemented method for determining a drug dose, comprising applying (S2) a machine learning algorithm (A1) and/or a rule-based algorithm (A2) to the pre-acquired cardiac current curve data (D) for classification of a deviation or conformity of at least one medical parameter (12) of the pre-acquired cardiac current curve data (D) from a norm, and in response to outputting the first class (C1), triggering (S4) a patient instruction (14) prompting the patient (11) to alter a drug dose in accordance to the classified deviation or conformity of the at least one medical parameter (12) of the pre-acquired cardiac current curve data (D) from the norm. In addition, the invention relates to a corresponding system and a method for providing a trained machine learning algorithm (A1).

## Description

The invention relates to a computer-implemented method for determining a drug dose. Furthermore, the invention relates to a computer-implemented method for providing a trained machine learning algorithm configured to determine a drug dose. In addition, the invention relates to a system for determining a drug dose.

A common treatment scenario in cardiology is the need to provide the patient with heart rate modulating drugs. This is necessary, for example, when administering beta-blockers to heart failure patients or antiarrhythmic drugs to control the rate of patients with persistent atrial fibrillation. Here, regular follow-up visits to a physician are necessary to adjust the drug dose accordingly and to avoid under- or over-therapy.

Moreover, in such patients, heart rate behavior is determined from long-term ECGs, exercise ECGs and/or manual evaluation of implant data in repeated outpatient follow-up examinations with subsequent manual dose adjustment.

Recording of a 24-hour long-term ECG or an exercise ECG requires the presence of the patient at the physician's office and is associated with relevant expenditure of time and personnel. Manual evaluation of implant data can be performed during a presence follow-up or remotely, but is also associated with relevant expenditure of time and personnel. Due to the effort involved, all three methods can only be performed at longer intervals and then only represent a snapshot in each case.

US 2010/0016746 A1 discloses a personal altering device and method of generating an alert of an event detected by an implanted diagnostic device. The method includes transmitting an event signal to the personal alerting device when one or more of the following occurs: ischemia is detected, a deviation from the baseline waveform is detected, a life-threatening trend begins, or symptoms are indicated. The method also includes generating an alarm signal from the personal alerting device, and the alarm signal can include one or more of a visual message, a light, a vibration, and a sound.

US 2020/0357513 A1 discloses techniques for remote monitoring of a patient and corresponding medical device(s). The remote monitoring comprises providing an interactive session configured to allow a user to navigate a plurality of sub sessions, determining a first set of data items in accordance with a first subsession, the first set of data items including the image data, determining a second set of data items in accordance with a second subsession of the interactive session, determining, based at least in part on the first set of data items and the second set of data items, an abnormality, and outputting a post-implant report of the interactive session.

Even though the above-mentioned methods assist the medical practitioner in timely diagnosing alterations in a patient condition, the physician is always involved in this communication. The physician must first evaluate patient data and diagnose a condition, then contact the patient, question him or her, and further make a recommendation as to whether the medication should be changed.

It is therefore an object of the present invention to provide an improved method for determining a drug dose capable of reducing a workload for the physician while accurately determining whether or not the drug dose of the patient needs to be changed in reaction to deviations in current cardiac current curve data.

The object is solved by a computer implemented method for determining a drug dose having the features of claim 1.

Furthermore, the object is solved by a computer-implemented method for providing a trained machine learning algorithm configured to determine a drug dose having the features of claim 13.

In addition, the object is solved by a system for determining a drug dose having the features of claim 14.

Further developments and advantageous embodiments are defined in the dependent claims.

The present invention provides a computer implemented method for determining a drug dose. The method comprises providing a first data set comprising cardiac current curve data of a patient acquired by an implantable medical device and applying a machine learning algorithm and/or a rule-based algorithm to the pre-acquired cardiac current curve data for classification of a deviation or conformity of at least one medical parameter of the pre-acquired cardiac current curve data from a norm.

Furthermore, method comprises outputting a second data set comprising at least a first class representing a deviation of the at least one medical parameter from the norm or a second class representing a conformity of at the least one medical parameter within a norm.

In addition, the method comprises, in response to outputting the first class or second class, triggering a patient instruction prompting the patient to alter a drug dose in accordance to the classified deviation or conformity of the at least one medical parameter of the pre-acquired cardiac current curve data from the norm.

Moreover, the present invention provides a computer implemented method for providing a trained machine learning algorithm configured to determine a drug dose.

The method comprises receiving a first training data set comprising first cardiac current curve data of a patient acquired by an implantable medical device and receiving a second training data set comprising at a first class representing a deviation of the at least one medical parameter from the norm and a second class representing a conformity of at the least one medical parameter within a norm.

Furthermore, method comprises training the first machine learning algorithm by an optimization algorithm which calculates an extreme value of a loss function for classification of the first class representing the deviation of the at least one medical parameter from the norm or the second class representing the conformity of at the least one medical parameter within a norm.

The present invention moreover provides a system for determining a drug dose. The system comprises means for providing a first data set comprising cardiac current curve data of a patient acquired by an implantable medical device.

The system further comprises a control unit configured to apply a machine learning algorithm and/or a rule-based algorithm to the pre-acquired cardiac current curve data for classification of a deviation or conformity of at least one medical parameter of the pre-acquired cardiac current curve data from a norm, the control unit being configured to output a second data set comprising at least a first class representing a deviation of the at least one medical parameter from the norm or a second class representing a conformity of at the least one medical parameter within a norm.

In addition, the system comprises means for, in response to outputting the first class, triggering a patient instruction prompting the patient to alter a drug dose in accordance to the classified deviation or conformity of the at least one medical parameter of the pre-acquired cardiac current curve data from the norm.

An idea of the present invention is to provide a method for regular recording and transmission of heart rate parameters and/or heart flow curves, which are transmitted to a central server. The server evaluates the transmitted data with regard to the heart rate behavior and/or to further information on the heart rhythm. According to rules adapted to the individual patient by the physician and/or a machine learning algorithm, the patient is automatically given feedback on the future medication dose.

The machine learning algorithm and/or rule-based algorithm thus checks whether the dose of the drug is still sufficient, or whether the patient should be instructed to increase or decrease the dose.

This is achieved by checking of the heart rate and/or checking of an ECG to determine whether drug induced side effects occur. For example, if a new AV block occurs or if an AV block deteriorates, the patient can be told to reduce the dose. If the heart rate goes up, for example, the patient can be asked if he or she is still taking the medication. If so, the dose should be increased.

The term AV Block refers to the conduction of cardiac excitation from the atria to the ventricles. There is the sinus node in the right atrium which acts as natural pacemaker. Its excitation is then brought via the atria to the AV node, which is the only physiological electrical connection between the atria and the ventricles. There, naturally, the excitation is delayed so that first the atria can contract and pump blood into the ventricles.

After a delay, the ventricles are excited which then continue to pump the blood, better filled by this atrial filling, into the body or lungs depending on the ventricle. AV conduction normally is between the beginning of the atrial excitation and the beginning of the QRS complex which is termed PQ time i.e. from the beginning of the P wave to a beginning of the QRS complex. The PQ time has normal values ranging from 120 to 200 ms.

Frequency modulating drugs can prolong the AV time to over 200 ms. This is termed a first degree AV block. In a second-degree AV block, for example only every third event is let through or every third event is blocked. In a third-degree AV block the drug completely suppresses AV conduction.

Machine learning algorithms are based on using statistical techniques to train a data processing system to perform a specific task without being explicitly programmed to do so. The goal of machine learning is to construct algorithms that can learn from data and make predictions. These algorithms create mathematical models that can be used, for example, to classify data or to solve regression type problems.

According to an aspect of the invention, the first class representing the deviation of the at least one medical parameter from the norm comprises a plurality of subclasses each representing a medical indication, in particular a drug induced side effect, a medical indication indicative of an insufficient drug dose and/or a medical indication indicative of an excessive drug dose. Thus, advantageously a plurality of different medical conditions can be detected based on the differences of the respective cardiac current curves.

According to a further aspect of the invention, the drug induced side effect, the medical indication indicative of an insufficient drug dose and/or the medical indication indicative of an excessive drug dose are an abnormal heart rate behavior and/or a conduction disturbance, in particular an atrioventricular block.

The triggering of the patient instruction prompting the patient to alter a drug dose in accordance with the classified deviation of the at least one medical parameter of the pre-acquired cardiac current curve data from the norm can thus effectively prevent the onset or worsening of the detected condition with minimal time delay.

According to a further aspect of the invention, the second class representing the conformity of at the least one medical parameter within a norm comprises changes in position, respiration and/or physical exertion. While changes in position, respiration and/or physical exertion of the patient can lead to changes in medical parameters, said changes are classified by the machine learning algorithm and/or the rule-based algorithm as not relevant, i.e. conforming to the norm.

According to a further aspect of the invention, the second data set further comprises a third class representing erroneous cardiac current curve data not suitable for application of the machine learning algorithm and/or the rule-based algorithm for classification of the deviation or conformity of at least one medical parameter of the pre-acquired cardiac current curve data from the norm. By classifying erroneous cardiac current curve data in a separate class, false classifications in the first and second classes can be advantageously prevented. According to a further aspect of the invention, the patient instruction is sent to a user communication device and/or smartphone, and wherein the patient instruction comprises information on a medically appropriate dose of the drug for the patient. The patient can thus validate the data set acquired by the implantable medical device by complying with said patient instruction.

According to a further aspect of the invention, the patient instruction is further prompting the patient to acknowledge reading said patient instruction, wherein if a reading confirmation is not sent to a server with a predetermined period, a notification is sent to a communication device of a health care provider. This advantageously notifies the healthcare provider that further action needs to be taken to inform the patient of a required change in drug dosage.

According to a further aspect of the invention, the notification, the patient instruction and the second data set outputted by the machine learning algorithm and/or the rule-based algorithm is stored on a central server and is accessible via a front-end application on the communication device, in particular a smart phone and/or a personal computer, of the health care provider. The healthcare provider can thus access all data relevant to a current medical condition of the patient via a convenient user interface.

According to a further aspect of the invention, the at least one medical parameter of the pre-acquired cardiac current curve data deviates from the norm if at least one numerical value of the pre-acquired cardiac current curve data is outside a predetermined range, exceeds or falls below a predetermined threshold value. By comparing the at least one numerical value of the pre-acquired cardiac current curve data to said predefined range, deviations in said cardiac current curve data are effectively identified.

According to a further aspect of the invention, the first data set comprises arrhythmia data, a heart rate, a patient activity, a chest impedance, a PQ time, an atrioventricular ratio, a heart rate daily profile, a heart rate at rest, a QRS width, and a heart rate variability and/or readings from electrodes or electrical contacts of the implantable medical device.

The machine learning algorithm and/or the rule-based algorithm thus uses a plurality of medical parameters and analyzes them in isolation and or in combination in order to identify deviations or conformity of the at least one medical parameter of the pre-acquired cardiac current curve data from a norm.

According to a further aspect of the invention, the cardiac current curve data is acquired by the implantable medical device at predetermined intervals and/or on request, and wherein the cardiac current curve data is transmitted to a central server via a patient communication device or smartphone. Said intervals can advantageously be set according to specific patient requirements and/or requirements set by a medical practitioner of the healthcare provider.

Furthermore, the rules of the rule-based algorithm are adaptable to the individual patient using prior cardiac current curve data of the patient. This way, said rules can advantageously be refined over time in order to be more relevant to a patient's individual health condition.

The herein described features of the system for determining a heart failure status of a patient are also disclosed for the computer implemented method for determining a heart failure status of a patient and vice versa.

For a more complete understanding of the present invention and advantages thereof, reference is now made to the following description taken in conjunction with the accompanying drawings. The invention is explained in more detail below using exemplary embodiments, which are specified in the schematic figures of the drawings, in which:
- Fig. 1: shows a flowchart of a computer implemented method and system for determining a drug dose according to a preferred embodiment of the invention; and
- Fig. 2: shows a flowchart of a computer implemented method for providing a trained machine learning algorithm A configured to determine a drug dose according to the preferred embodiment of the invention.

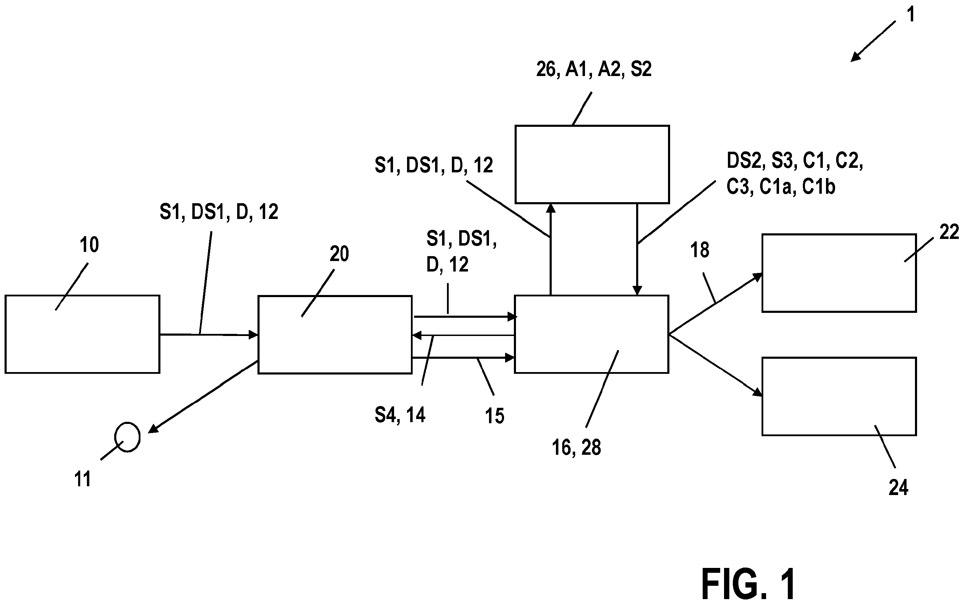

The system 1 shown in Fig. 1 comprises means for providing S1 a first data set DS1 comprising cardiac current curve data D of a patient 11 acquired by an implantable medical device 10.

The system further comprises a control unit configured to apply S2 a machine learning algorithm A1 and/or a rule-based algorithm A2 to the pre-acquired cardiac current curve data D for classification of a deviation or conformity of at least one medical parameter 12 of the pre-acquired cardiac current curve data D from a norm, the control unit being configured to output S3 a second data set DS2 comprising at least a first class C1 representing a deviation of the at least one medical parameter 12 from the norm or a second class C2 representing a conformity of at the least one medical parameter 12 within a norm. The parameter 12 may also be generated by the implantable medical device or by any other component, including especially the A1 and/or A2.

In addition, the system comprises means for, in response to outputting the first class C1, triggering S4 a patient instruction 14 prompting the patient 11 to alter a drug dose in accordance to the classified deviation or conformity of the at least one medical parameter 12 of the pre-acquired cardiac current curve data D from the norm.

The first class C1 representing the deviation of the at least one medical parameter 12 from the norm comprises a plurality of subclasses C1a, C1b each representing a medical indication, in particular a drug induced side effect, a medical indication indicative of an insufficient drug dose and/or a medical indication indicative of an excessive drug dose.

The drug induced side effect, the medical indication indicative of an insufficient drug dose and/or the medical indication indicative of an excessive drug dose are an abnormal heart rate behavior and/or a conduction disturbance, in particular an atrioventricular block. Furthermore, the second class C2 representing the conformity of at the least one medical parameter 12 within a norm comprises changes in position, respiration and/or physical exertion.

The second data set DS2 further comprises a third class C3 representing erroneous cardiac current curve data not suitable for application of the machine learning algorithm A1 and/or the rule-based algorithm A2 for classification of the deviation or conformity of at least one medical parameter 12 of the pre-acquired cardiac current curve data D from the norm.

The patient instruction 14 is sent to a user communication device 20 and/or smartphone. Moreover, the patient instruction 14 comprises information on a medically appropriate dose of the drug for the patient 11.

The patient instruction 14 is further prompting the patient 11 to acknowledge reading said patient instruction 14, wherein if a reading confirmation 15 is not sent to a server 16 with a predetermined period, a notification 18 is sent to a communication device 22 of a health care provider.

The notification 18, the patient instruction 14 and the second data set DS2 outputted by the machine learning algorithm A1 and/or the rule-based algorithm A2 is stored on a central server 16 and is accessible via a front-end application 24 on the communication device 22, in particular a smart phone and/or a personal computer, of the health care provider.

The at least one medical parameter 12 of the pre-acquired cardiac current curve data D deviates from the norm if at least one numerical value of the pre-acquired cardiac current curve data D is outside a predetermined range, exceeds or falls below a predetermined threshold value.

The first data set DS1 comprises arrhythmia data, a heart rate, a patient activity, a chest impedance, a PQ time, an atrioventricular ratio, a heart rate daily profile, a heart rate at rest, a QRS width, a heart rate variability and/or readings from electrodes or electrical contacts of the implantable medical device.

The cardiac current curve data D is acquired by the implantable medical device 10 at predetermined intervals and/or on request, and wherein the cardiac current curve data is transmitted to a central server 16 via a patient communication device or smartphone. Rules of the rule-based algorithm A2 are adaptable to the individual patient 11 using prior cardiac current curve data D of the patient 11.

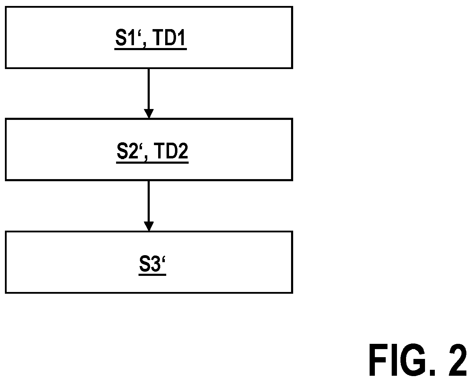

Fig. 2 shows a flowchart of a computer implemented method for providing a trained machine learning algorithm A configured to determine a drug dose according to the preferred embodiment of the invention.

The method comprises receiving S1' a first training data set TD1 comprising first cardiac current curve data D of a patient 11 acquired by an implantable medical device 10. Moreover, the method comprises receiving S2' a second training data set TD2 comprising at a first class C1 representing a deviation of the at least one medical parameter 12 from the norm and a second class C2 representing a conformity of at the least one medical parameter 12 within a norm.

In addition, the method comprises training S3' the first machine learning algorithm A1 by an optimization algorithm which calculates an extreme value of a loss function for classification of the first class representing the deviation of the at least one medical parameter 12 from the norm or the second class C2 representing the conformity of at the least one medical parameter 12 within a norm.

### Reference Signs

- 1: system
- 10: implantable medical device
- 11: patient
- 12: medical parameter
- 14: patient instruction
- 15: reading confirmation
- 16: central server
- 18: notification
- 20: patient communication device
- 22: communication device of a healthcare provider
- 24: front-end application
- 26: control unit
- 28: means
- A1: machine learning algorithm
- A2: rule-based algorithm
- C1: first class
- C2: second class
- C3: third class
- C1a, C1b: subclasses
- D: cardiac current curve data
- DS1: first data set
- DS2: second data set
- S1-S4: method steps
- S1'-S3': training method steps
- TD1: first training data set
- TD2: second training data set

## Claims

1. Computer implemented method for determining a drug dose, comprising the steps of:
providing (S1) a first data set (DS1) comprising cardiac current curve data (D) of a patient (11) acquired by an implantable medical device (10);
applying (S2) a machine learning algorithm (A1) and/or a rule-based algorithm (A2) to the pre-acquired cardiac current curve data (D) for classification of a deviation or
conformity of at least one medical parameter (12) of the pre-acquired cardiac current curve data (D) from a norm;
outputting (S3) a second data set (DS2) comprising at least a first class (C1) representing a deviation of the at least one medical parameter (12) from the norm or a second class (C2) representing a conformity of at the least one medical parameter (12) within the norm;
in response to outputting the first class (C1), triggering (S4) a patient instruction (14) prompting the patient (11) to alter a drug dose in accordance to the classified deviation or conformity of the at least one medical parameter (12) of the pre-acquired cardiac current curve data (D) from the norm.

2. Computer implemented method of claim 1, wherein the first class (C1) representing the deviation of the at least one medical parameter (12) from the norm comprises a plurality of subclasses (C1a, C1b) each representing a medical indication, in particular a drug induced side effect, a medical indication indicative of an insufficient drug dose and/or a medical indication indicative of an excessive drug dose.

3. Computer implemented method of claim 2, wherein the drug induced side effect, the medical indication indicative of an insufficient drug dose and/or the medical indication indicative of an excessive drug dose are an abnormal heart rate behavior and/or a conduction disturbance, in particular an atrioventricular block.

4. Computer implemented method of any one of the preceding claims, wherein the second class (C2) representing the conformity of at the least one medical parameter (12) within a norm comprises changes in position, respiration and/or physical exertion.

5. Computer implemented method of any one of the preceding claims, wherein the second data set (DS2) further comprises a third class (C3) representing erroneous cardiac current curve data not suitable for application of the machine learning algorithm (A1) and/or the rule-based algorithm (A2) for classification of the deviation or conformity of at least one medical parameter (12) of the pre-acquired cardiac current curve data (D) from the norm.

6. Computer implemented method of any one of the preceding claims, wherein the cardiac current curve data (D) is acquired by the implantable medical device (10) at predetermined intervals and/or on request, and wherein the cardiac current curve data (D) is transmitted to a central server (16) via a patient communication device (20) or smartphone.

7. Computer implemented method of claim 6, wherein the patient instruction (14) is sent to a user communication device (20) and/or smartphone, and wherein the patient instruction (14) comprises information on a medically appropriate dose of the drug for the patient (11).

8. Computer implemented method of claim 6, wherein the patient instruction (14) is further prompting the patient (11) to acknowledge reading said patient instruction (14), wherein if a reading confirmation (15) is not sent to the central server (16) with a predetermined period, a notification (18) is sent to a communication device (22) of a health care provider.

9. Computer implemented method of claim 8, wherein the notification (18), the patient (11) instruction (14) and the second data set (DS2) outputted by the machine learning algorithm (A1) and/or the rule-based algorithm (A2) is stored on the central server (16) and is accessible via a front-end application (24) on the communication device (22), in particular a smart phone and/or a personal computer, of the health care provider.

10. Computer implemented method of any one of the preceding claims, wherein the at least one medical parameter (12) of the pre-acquired cardiac current curve data (D) deviates from the norm if at least one numerical value of the pre-acquired cardiac current curve data (D) is outside a predetermined range, exceeds or falls below a predetermined threshold value.

11. Computer implemented method of any one of the preceding claims, wherein the first data set (DS1) comprises arrhythmia data, a heart rate, a patient activity, a chest impedance, a PQ time, an atrioventricular ratio, a heart rate daily profile, a heart rate at rest, a QRS width, a heart rate variability and/or readings from electrodes or electrical contacts of the implantable medical device (10).

12. Computer implemented method of any one of the preceding claims, wherein rules of the rule-based algorithm (A2) are adaptable to the individual patient (11) using prior cardiac current curve data (D) of the patient (11).

13. Computer-implemented method for providing a trained machine learning algorithm (A1) configured to determine a drug dose, comprising the steps of:
receiving (S1') a first training data set (TD1) comprising first cardiac current curve data (D) of a patient (11) acquired by an implantable medical device (10);
receiving (S2') a second training data set (TD2) comprising at a first class (C1) representing a deviation of the at least one medical parameter (12) from the norm and
a second class (C2) representing a conformity of at the least one medical parameter (12) within a norm; and
training (S3') the machine learning algorithm (A1) by an optimization algorithm which calculates an extreme value of a loss function for classification of the first class representing the deviation of the at least one medical parameter (12) from the norm or
the second class (C2) representing the conformity of at the least one medical parameter (12) within a norm.

14. System (1) for determining a drug dose, comprising:
an implantable medical device (10) for providing a first data set (DS1) comprising cardiac current curve data (D) of a patient (11);
a control unit (26) configured to apply a machine learning algorithm (A1) and/or a rule-based algorithm (A2) to the pre-acquired cardiac current curve data (D) for classification of a deviation or conformity of at least one medical parameter (12) of the pre-acquired cardiac current curve data (D) from a norm, the control unit (26) being configured to output a second data set (DS2) comprising at least a first class (C1) representing a deviation of the at least one medical parameter (12) from the norm or a second class (C2) representing a conformity of at the least one medical parameter (12) within a norm; and
means (28) for, in response to outputting the first class (C1), triggering (S4) a patient instruction (14) prompting the patient (11) to alter a drug dose in accordance to the classified deviation or conformity of the at least one medical parameter (12) of the pre-acquired cardiac current curve data (D) from the norm.

15. Computer program with program code to perform the method of any one of claims 1 to 12 when the computer program is executed on a computer.
